# EUROPEAN PATENT SPECIFICATION

(11) **EP 0 416 609 B1**
(45) Date of publication and mention of the grant of the patent: **08.01.1997**
(21) Application number: 90117171.0
(22) Date of filing: 06.09.1990
(51) Int. Cl.: C07D 333/58, A61K 31/38

(54) **Benzo[b]thiophene-containing lipoxygenase-inhibiting compounds**
Benzo[b]thiophen enthaltende lipoxygenaseinhibierende Verbindungen
Composés inhibiteurs de lipoxygénase dérivés du benzo[b]thiophène

(30) Priority: 07.09.1989 US 404300; 28.08.1990 US 572451
(43) Date of publication of application: 13.03.1991
(73) Proprietor: ABBOTT LABORATORIES, Abbott Park, Illinois 60064-3500 (US)
(72) Inventor: Brooks, Dee W., Libertyville, IL 60048 (US); Summers, James B., Libertyville, IL 60048 (US)
(74) Representative: Modiano, Guido, Dr.-Ing.

(56) References cited:
- EP-A- 0 279 263
- EP-A- 0 320 628
- EP-A- 0 346 939

## Description

### Technical Field

This invention relates to pro-drug derivatives of lipoxygenase-inhibiting compounds having metabolically cleavable groups. It also relates to compositions for inhibiting lipoxygenase enzymes in human and animal hosts in need of such treatment.

### Background of the Invention

The lipoxygenases are a family of enzymes which catalyze the oxygenation of arachidonic acid. The enzyme 5-lipoxygenase converts arachidonic acid to 5-hydroperoxyeicosatetraenoic acid (5-HPETE). This is the first step in the metabolic pathway yielding S-hydroxyeicosatetraenoic acid (5-HETE) and the important class of mediators, the leukotrienes (LTs).

Similarly, 12- and 15-lipoxygenase, convert arachidonic acid to 12- and 15-HPETE, respectively. Biochemical reduction of 12-HPETE leads to 12-HETE, while 15-HPETE is the precursor of the class of biological agents known as the lipoxins.

A variety of biological effects are associated with these products from lipoxygenase metabolism of arachidonic acid and they have been implicated as mediators in various disease states. For example, the leukotrienes LTC₄ and LTD₄ are potent constrictors of human airways *in vitro,* and aerosol administration of these substances to non-asthmatic volunteers induces broncho-constriction. LTB₄ and 5-HETE are potent chemotactic factors for inflammatory cells such as polymorphonuclear leukocytes. They also have been found in the synovial fluid of rheumatoid arthritic patients. Leukotrienes have also been implicated as important mediators in asthma, rheumatoid arthritis, gout, psoriasis, allergic rhinitis, adult respiratory distress syndrome, Crohn's disease, endotoxin shock, inflammatory bowel disease and/or ischemia - induced myocardial or brain injury, among others. The biological activity of the leukotrienes has been reviewed by Lewis and Austen (J. Clinical Invest. **73**: 889 (1984)) and by J. Sirois (Adv. Lipid Res. **21**: 78 (1985)).

The product 12-HETE has been found in high levels in epidermal tissue of patients with psoriasis. The lipoxins have recently been shown to stimulate elastase and superoxide ion release from neutrophils. Thus, lipoxygenase enzymes are believed to play an important role in the biosynthesis of mediators of asthma, allergy, arthritis, psoriasis, and inflammation. It is postulated that interrupting the biochemical pathways involved in the vanous manifestations of these disease states will provide effective systemic andlor symptomatic treatment of these diseases.

It is therefore an object of this invention to provide prodrug derivatives of lipoxygenase inhibitors having an added, metabolically cleavable group. By the removal of their cleavable groups, these prodrugs are converted *in vivo* to active lipoxygenase inhibitors. Such prodrugs have been shown to be useful, for example, in improving the bioavailability of pharmaceuticals by enhancing their solubility, stability, and/or rate of absorption.

### Summary of the Invention

In accordance with the above objects, the present invention provides, in one aspect, 5- and/or 12-lipoxygenase-inhibiting compounds selected from the group consisting of
N-ethoxycarbonyloxy-N-[1-(benzo[b]thien-2-yl)ethyl] urea;
N-methoxycarbonyloxy-N-[1-(benzo[b]thien-2-yl)ethyl] urea;
N-tert-butoxycarbonyloxy-N-[1-(benzo[b]thien-2-yl)ethyl]urea;
N-thioethylcarbonyloxy-N-[1-(benzo[b]thien-2-yl)ethyl] urea;
N-glutaryloxy-N-[1-(benzo[b]thien-2-yl)ethyl] urea; and
N.succinyloxy-N-[1-(benzo[b]thien-2-yl)ethyl] urea;

### Detailed Description

Certain compounds of the present invention contain one or more asymmetric carbon atoms, giving rise to enantiomeric and diastereomeric forms of the compounds.

It is to be understood that the invention encompasses the enantiomers and di astereomers as well as mixtures thereof including racemic mixtures.

The term "metabolically cleavable group" as used herein denotes a moiety which is readily cleaved *in vivo* from the compound to which it is attached, which compound (after cleavage) remains or becomes physiologically active.

The metabolically cleavable groups of the above compounds are, respectively, ethoxycarbonyl, methoxycarbonyl, tert-butoxycarbonyl, thioethylcarbonyl, glutaryl and succinyl.

Because of the ease with which the metabolically cleavable groups of the inventive compounds are removed *in vivo,* these compounds may act as prodrugs of other lipoxygenase inhibitors. The inventive compounds therefore have the advantage that, in addition to themselves being active inhibitors of lipoxygenase enzymes, they are converted*in vivo* to active lipoxygenase-inhibiting residues. Moreover, as prodrugs the compounds of the present invention may exhibit improved bioavailability as a result of enhanced solubility and/or rate of absorbtion.

The compounds of the invention inhibit lipoxygenase activity, which makes the compounds useful in the treatment and prevention of disease states in which lipoxygenase may be involved, including, but not limited to, asthma, rheumatoid arthritis, gout, psoriasis, allergic rhinitis, allergic dermatitis, inflammatory disorders of the skin, acne, adult respiratory distress syndrome, Crohn's disease, endotoxin shock, inflammatory bowel disease and/or ischemia-induced myocardial or brain injury. The compounds of the invention inhibit oxidative modification of lipids which makes them useful in diseases such as atherosclerosis. In some cases this will involve preventing the underlying cause of the disease state and in other cases, while the underlying disease will not be affected, the compounds of this invention will have the benefit of ameliorating the symptoms or preventing the manifestations of the disease.

Accordingly, this invention provides the use of the above compounds for manufacturing a medicament for inhibiting 5- and/or 12-lipoxygenase activity in a human or lower animal host in need of such treatment. The compounds are administered to the human or lower animal host in a therapeutically effective amount to inhibit lipoxygenase activity in the host. The compounds of the invention may be used for treating asthma, rheumatoid arthritis, gout, psoriasis, allergic rhinitis, allergic dermatitis, inflammatory disorders of the skin, acne, atherosclerosis, adult respiratory distress syndrome, Crohn's disease, endotoxin shock,inflammatory bowel disease and/or ischemia-induced myocardial or brain injury in a human or lower animal in need of such treatment.

Further, the compounds of the invention may be used for treating or preventing the symptoms of the disease states mentioned above.

The compounds of the present invention may be administered orally, parenterally or topically in dosage unit formulations containing conventional nontoxic pharmaceutically acceptable carriers, adjuvants and vehicles as desired.

The term "parenterally" as used herein includes subcutaneous, intravenous, intra-arterial injection or infusion techniques, without limitation. The term "topically" encompasses administration rectally and by inhalation spray, as well as by the more common routes of the skin and the mucous membranes of the mouth and nose.

Total daily dose of the compounds of this invention administered to a host in single or divided doses may be in amounts, for example, of from 0.001 to 100 mg/kg body weight daily and more usually 0.1 to 35 mg/kg/day. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, rates of absorption and excretion, combination with other drugs and the severity of the particular disease being treated.

### Formulation of Pharmaceutical Composition

This invention also provides pharmaceutical compositions in unit dosage form for the inhibition of 5- or 12-lipoxygenase activity in a human or lower animal host in need of such treatment, comprising a compound of this invention and one or more nontoxic pharmaceutically acceptable carriers, adjuvants or vehicles. The amount of active ingredient that may be combined with such materials to produce a single dosage form will vary depending upon various factors, as indicated above.

A variety of materials can be used as carriers, adjuvants and vehicles in the composition of this invention, as available in the pharmaceutical arts. Injectable preparations, such as oleaginous solutions, suspensions or emulsions, may be formulated according to known art, using suitable dispersing or wetting agents and suspending agents, as needed. The sterile injectable preparation may employ a nontoxic parenterally acceptable diluent or solvent as, for example, sterile nonpyrogenic water or 1,3-butanediol.

Among the other acceptable vehicles and solvents that may be employed are 5% dextrose injection, Ringer's injection and isotonic sodium chloride injection (as described in the USP/NF). In addition, sterile, fixed oils are conventionally employed as solvents or suspending media. For this purpose any bland fixed oil may be used, including synthetic mono-, di- or triglycerides. Fatty acids such as oleic acid can also be used in the preparation of injectable compositions.

Suppositories for rectal administration of the compound of this invention can be prepared by mixing the drug with suitable nonirritating excipient such as cocoa butter and polyethylene glycols, which are solid at ordinary temperatures but liquid at body temperature and which therefore melt in the rectum and release the drug.

Solid dosage forms for oral administration include capsules, tablets, pills, troches, lozenges, powders and granules. In such solid dosage forms, the active compound may be admixed with at least one inert diluent such as sucrose, lactose or starch. Such dosage forms may also comprise, as is normal practice, pharmaceutical adjuvant substances, e.g., stearate lubricating agents. In the case of capsules, tablets and pills, the dosage forms may also comprise buffering agents. Solid oral preparations can also be prepared with enteric or other coatings which modulate release of the active ingredients. Liquid dosage forms for oral administration include pharmaceutically acceptable emulsions, solutions, suspensions, syrups and elixirs containing inert nontoxic diluents commonly used in the art, such as water and alcohol. Such compositions may also comprise adjuvants, such as wetting agents, emulsifying suspending, sweetening, flavoring and perfuming agents.

### Synthesis of the Compounds

Several synthetic methods may be used to prepare compounds of this invention. Some of the methods for preparing intermediates are described by schemes 1-3 below.

An intermediate hydroxylamine **3**, which is used in preparing further intermediates according to Schemes 2 and 3, may be prepared as described in Scheme 1. 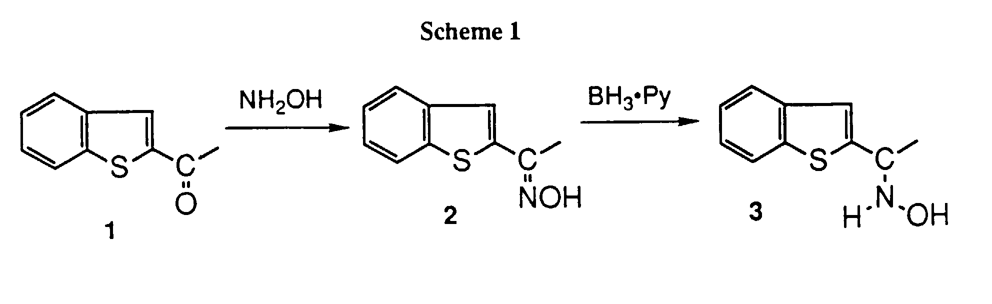 In scheme 1, 2-acetyl benzo[b]thiophene, **1**, is treated with hydroxylamine in ethanol/pyridine to produce the oxime **2**. This is reduced to the hydroxylamine **3** with borane pyridine complex .

An intermediate for preparing compounds of the invention can be prepared according to the method outlined in scheme 2, below (see Example 2, method (b)). 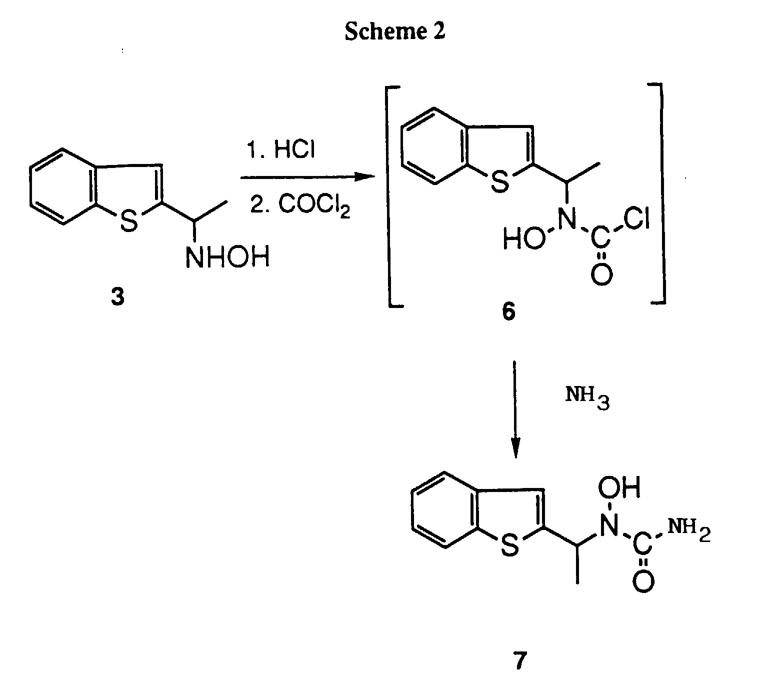 Hydroxylamine **3**, the synthesis of which was described above, is treated with gaseous HCl followed by phosgene. The resulting putative carbamoyl chloride **6** is reacted without isolation with aqueous ammonia to yield the urea **7**.

Compounds of the invention can be prepared by way of the intermediate urea compound **7** below (see Example 2, method (a)). The preparation of the compound **7** is shown in Scheme 3 below where R³ is a metabolically cleavable group. 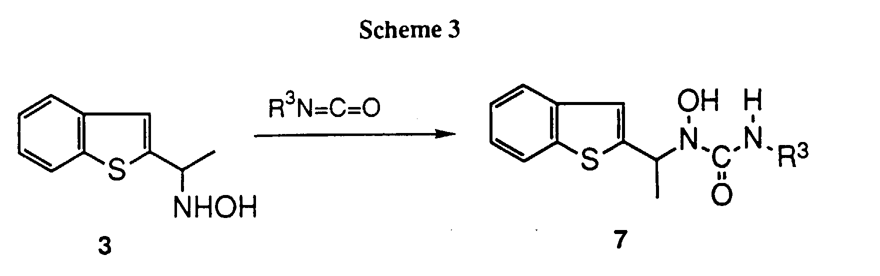 Hydroxylamine **3** is treated with an isocyanate (R³NCO), followed by ammonium chloride workup to give the urea **7**.

### Example 1 (intermediate)

### (1-Benzo[b]thien-2-ylethyl)hydroxylamine

### a. 2-Acetyl benzo[b]thiophene.

*Method a.* Using the method described in Scheme 1, benzo[b]thiophene (10 g, 75 mmole) was dissolved in THE (50 mL) and cooled to -78°C. Butyl lithium (28 mL, 2.7 M in hexanes) was added. The mixture was stirred for 15 minutes and N,O-dimethyl acetohydroxamic acid was added. Following an additional 30 minutes of stirring, the reaction was quenched at -78°C with ethanol and 2N HCl solution and extracted into ether. The solvent was removed *in vacuo* and the residue chromatographed on silica gel eluting with 20% ether in pentane to yield 6.9 g of the desired product as a white solid.
*Method b.* To a solution of benzo[b]thiophene (10.0 g, 75 mmole) in THE (50 mL) was added n-butyl lithium (33 mL, 2.5M in hexanes) at -70°C under N₂ The mixture, containing a white precipitate, was stirred at -70°C for 1 hour. Acetaldehyde (4.6 mL, 82 mmole) was added dropwise. After a few minutes the reaction was quenched with saturated NH₄Cl solution. The layers were separated, the organic layer dried over MgSO₄, filtered, and evaporated to give a white solid (10 g) which was used directly for the next step.

The alcohol prepared as described above (1.0 g) in acetone (50 mL) was cooled to 5°C and Jones Reagent was added dropwise until the orange yellow color persisted (1.4 mL). The reaction mixture was diluted with water and the desired product precipitated. It was collected by filtration to give 0.85 g.

b. 2-Acetyl benzo[b]thiophene oxime. 2-Acetyl benzo[b]thiophene (5 g, 28.4 mmole), prepared as described in step a above, and hydroxylamine hydrochloride 3.0 g, 42.6 mmole) were dissolved in a mixture of ethanol (50 mL) and pyridine (50 mL) and allowed to stir at room temperature for 2 hours. Most of the solvent was removed *in vacuo* and the residue dissolved in ether. After washing with 2N HCl (100 mL), the solution was dried over MgSO₄ and evaporated. A white crystalline solid was obtained and was carried on without further purification.

An alternative work-up was also used. The reaction mixture was diluted with water (300 mL) and the product precipitated. It was filtered off and dried *in vacuo.*

c. 1-Benzo[b]thien-2-ylethyl hydroxylamine. The oxime prepared as in step b above (3.5 g, 18.5 mmole) was dissolved in ethanol (25 mL) and cooled to 0°C. Borane pyridine complex (3.7 mL, 37 mmole) was added via syringe under nitrogen followed ten minutes later by 20% HCl in ethanol (30 mL). Within thirty minutes the reaction was complete and was brought to pH 9 with the addition of solid sodium carbonate or 2N NaOH. The mixture was extracted into ether and dried over MgS0₄. After evaporation a white solid (3.0 g) was obtained. This was carried on without further purification.

### Example 2 (intermediate)

### -N-hydroxy-N-(1-benzo[b]thien-2-ylethyl)urea

*Method a.* Using the method of Scheme 3, 1-benzo[b]thien-2-ylethyl hydroxylamine prepared as described in Example 1, step c (2.0 g, 10 mmole), was refluxed for thirty minutes with trimethylsilyl isocyanate (1.65, 14.2 mmole) in dioxane (30 mL). The reaction mixture was then washed with saturated NH4Cl solution, dried with MgS0_{4,} and evaporated.
*Method b.* Using the method of Scheme 2, 1-benzo[b]thien-2-ylethyl hydroxylamine prepared as described in example 1, step c, was dissolved in toluene (100 mL) and HCl gas was bubbled through the mixture at a moderate rate for about four minutes. The solution was then heated to reflux and phosgene was bubbled through for another four minutes. After an additional one hour reflux, the mixture was allowed to cool to room temperature and then added to excess cold ammonium hydroxide solution. The precipitate was collected and recrystallized.

MeltingPoint: 157-158°C.
NMR (300 MHz, DMSO-d6): 1.51 (d, 3H); 5.55 (q, 1H); 6.45 (brs, 2H), 7.25-7.37 (m, 3H); 7.75-7.91 (m, 2H); 9.35 (s, 1H).
Mass spectrum (CI-NH3): 237 (M+1)+, 221,194,176,161.

### Example 3 (reference example)

### N-acetoxy-N-[1-(benzo[b]thien-2-yl)ethyl]urea

To a stirred solution of N-hydroxy-N-1-(benzo[b]thien-2ylethyl) urea (product of Example 2, 0.80 g, 3.4 mmol) in THE (20 mL) at 0°C was added triethylamine (0.52 mL, 3.7 mmol) followed by acetyl chloride (0.27 mL, 3.7 mmol). The reaction stirred until complete before quenching with water. The layers were separated and the aqueous layer was extracted with ethyl acetate (3 x 50 mL). The organics were combined, dried, and evaporated. The solid obtained was recrystallized to constant melting point using ethyl acetate:hexane. The title compound was prepared in a 68% yield.
M.P.=138°C; 1H
NMR (300 MHz, DMSO-d6);
   1.50 (3H, d), 2.12 (3H, br.s), 5.69 (1H, m), 6.86 (2H, br.s), 7.34 (3H, m), 7.80 (1H, m), 7.91 (1H, m); MS (M+NH4)+= 296, (M+H)+= 279.

### Example 4

### N-ethoxycarbonyloxy-N-[1-(benzo[b]thien-2-yl)ethyl]urea

To a solution of N-hydroxy-N-(benzo[b]thien-2ylethyl) urea (product of Example 2, 2.00 g, 8.5 mmol) and triethylamine (944 mg, 9.35 mmol) in CH₂Cl₂ (40 mL), was added dropwise ethylchloroformate (1.01 g, 9.35 mmol). Upon completion of addition, the reaction was stirred for 10 min. It was then diluted with brine (40 mL) and the layers were separated. The aqueous was extracted with CH₂Cl₂ (2x 40 mL). The organics were combined, dried with MgS04 and concentrated. The resulting residue was crystallized in ethylacetate/hexanes to afford the desired product as a white solid.
M.P.=140-141°C; 1H
NMR (300 MHz, DMSO-d6): 0.92-1.34 (bm, 3H), 1.54 (d, 3H, J = 7.5 Hz), 3.92-4.34 (m, 2H), 5.70 (m, 1H), 6.98 (bs, 2H), 7.29-7.39 (m, 3H), 7.81 (m, 1H), 7.91 (m, 1H);
Mass spectrum (M+H)+=309.
Analysis calc'd for C₁₄H₁₆N₂O₄S : C=54.53, H=5.23, N=9.09; Found: C=54.20, H=5.16, N=9.07.

### Example 5

### N-methoxycarbonyloxy-N-[1-(benzo[b]thien-2-yl)ethyl]urea

The desired material was prepared according to the procdure of Example 4 substituting methyl chloroformate for ethyl chloroformate.
M.P.=124-125°C.
NMR (300 MHz, DMSO-d6): 1.53 (d, 3H, J = 7 Hz), 3.78 (m, 3H), 5.70 (m, 1H), 6.99 (bs, 2H), 7.35 (m, 3H), 7.81 (m, 1H), 7.91 (m, 1H).
Mass spectrum )M+H)+=295.
Analysis calc'd for C₁₃H₄N₂O₄S: C=53.05, H=4.79, N=9.52; Found: C=53.13, H= 4.79, N=9.51.

### Example 6

### N-ten-butoxycarbonyloxy-N-[1-(benzo[b]thien-2-yl)ethyl]urea

The desired material was prepared according to the procedure of Example 4 substituting di-tert-butylcarbonate for ethyl chloroformate.
M.P.=120-122°C.
NMR (300 MHz, DMSO-d6): 1.10-1.49 (m, 9H), 1.52 (d, 3H, J = 7 Hz), 5.69 (m, 1H), 6.89 (bs, 2H), 7.34 (m, 3H), 7.81 (m, 1H), 7.91 (m, 1H).
Mass spectrum: (M+H)+=337.
Analysis calc'd for C₁6H₂₀N₂O₄S: C=57.12, H=5.99, N=8.33; Found: C=57.14, H=6.04, N=8.30. '

### Example 7

### N-thioethylcarbonyloxy-N-[1-(benzo[b]thien-2-yl)ethyl]urea

The desired material was prepared according to the procedure of Example 4 substituting ethyl chlorothioformate for ethyl chloroformate.
M.P.=130-132°C.
NMR (300 MHz, DMSO-d6): 0.70-1.33 (m, 3H), 1.58 (d, 3H, J = 7 Hz), 2.59-2.97 (m, 2H), 5.69 (m, 1H), 7.10 (bs, 2H), 7.29-7.40 (m, 3H), 7.81 (m, 1H), 7.93 (m, 1H).
Mass spectrum: (M+H)+=325.
Analysis calc'd for C₁₄H₁₆N₂O₃S₂: C=51.83, H=4.97, N=8.64; Found: C=52.01, H=4.97, N=8.68.

### Example 8

### N-glutaryloxy-N-[1-(benzo[b]thien-2-yl)ethyl]urea

The desired material was prepared according to the procedure of Example 3 substituting glutaric anhydride for acetyl chloride.
M.P.= 129.5-130.5°C.
NMR (300 MHz, DMSO-d6): 1.49 (d, 3H, J = 7 Hz), 1.74 (m, 2H), 2.23 (m, 2H), 2.51 (under DMSO-2H), 5.69 (m, 2H), 6.86 (bs, 2H), 7.35 (m, 3H), 7.80 (m, 1H), 7.91 (m, 1H), 12.13 (bs, 1H).
Mass spectrum: (M+H)+=351.
Analysis calc'd for C₁₆H₁₈N₂O₅S: C=54.84, 1-1=5.18, N=8.00; Found: C=54.71, H=5.29, N=7.91.

### Example 9

### N-succinyloxy,-N-[1-(benzo[b]thien-2-yl)ethyl]urea

The desired material was prepared according to the procedure of Example 3 substituting succinic anhydride for acetyl chloride.
M.P. 127.5-129.0°C.
NMR (300 MHz, DMSO-d6): 1.51 (d, 3H, J = 7 Hz), 2.54 (m, 2H), 2.69 (m, 2H), 5.71 (m, lH), 6.81 (bs, 20 2H), 7.35 (m, 3H), 7.80 (m, 1H), 7.91 (m, 1H), 12.45 (bs, 1H).
Mass spectrum: (M+H)+=337.
Analysis calc'd for C₁₅H₁₆N₂O₅S: C=53.56, H=4.80, N=8.33; Found: C=53.37, H=4.96, N=8.33.

### Lipoxygenase IC₅₀ Determination

Assays to determine S-lipoxygenase inhibitory activity were performed in 200 microL incubations containing the 20,000xg supernatant from 6x104 homogenized RBL-1 cells, 2% DMSO vehicle and various concentrations of the test compound. Reactions were initiated by addition of radiolabelled arachidonic acid and terminated by acidification and ether extraction. Reaction products were separated from nonconverted substrate by thin layer chromatography and measured by liquid scintillation spectroscopy. All treatments were evaluated in triplicate incubations. Inhibition of S-lipoxygenase activity was computed by comparison of the quantity of products formed in the treatment incubations to the mean product formation in vehicle control groups (n=8). ICso values and 95% confidence limits were computed by linear regression analysis of percentage inhibition versus log inhibitor concentration plots. Inhibitory potencies for representative examples of compounds of this invention are listed in Table 1.

**Table 1**

| *In vitro* 5-lipoxygenase Inhibitory Potency of Compounds of this Invention. | |
|---|---|
| Ex. | IC₅₀(µM) |
| | |
| 4 | 1.5 |
| 5 | 1.2 |
| 9 | 0.6 |
| | |

## Claims (Claims for the following Contracting State(s): AT, BE, CH, LI, DE, DK, FR, GB, IT, LU, NL, SE)

1. A compound selected from the group consisting of
N-ethoxycarbonyloxy-N-[1-(benzo[b]thien-2-yl)ethyl]urea;
N-methoxycarbonyloxy-N-[1-(benzo[b]thien-2-yl)ethyl]urea;
N-tert-butoxycarbonyloxy-N-[1-(benzo[b]thien-2-yl)ethyl]urea;
N-thioethylcarbonyloxy-N-[1-(benzo[b]thien-2-yl)ethyl]urea;
N-glutaryloxy-N-[1-(benzo[b]thien-2-yl)ethyl]urea; and
N-succinyloxy-N-[1-(benzo[b]thien-2-yl)ethyl]urea.

2. A pharmaceutical composition for inhibiting lipoxygenase enzymes comprising a therapeutically effective amount of a compound as defined by Claim 1 in combination with a pharmaceutically acceptable carrier.

3. A compound according to Claim 1 for use in inhibiting lipoxygenase enzymes in a host mammal in need of such treatment.

4. Use of a compound according to Claim 1 for manufacturing a medicament for inhibiting lipoxygenase enzymes in a host mammal in need of such treatment.

## Claims (Claims for the following Contracting State(s): ES)

1. A process for preparing a compound selected from the group consisting of
a) N-ethoxycarbonyloxy-N-[1-(benzo[b]thien-2-yl)ethyl]urea;
b) N-methoxycarbonyloxy-N-[1-(benzo[b]thien-2-yl)ethyl]urea;
c) N-tert-butoxycarbonyloxy-N-[1-(benzo[b]thien-2-yl)ethyl]urea;
d) N-thioethylcarbonyloxy-N-[1-(benzo[b]thien-2-yl)ethyl]urea;
e) N-glutaryloxy-N-[1-(benzo[b]thien-2-yl)ethyl]urea; and
f) N-succinyloxy-N-[1-(benzo[b]thien-2-yl)ethyl]urea;
comprising reacting N-hydroxy-N-[1-(benzo[b]thien-2-yl)ethyl]urea with an N-hydroxy blocking group reagent which is
ethylchloroformate in the case of a) above;
methylchloroformate in the case of b) above;
di-tert-butylcarbonate in the case of c) above;
ethyl chlorothioformate in the case of d) above;
glutaric anhydride in the case of e) above; and
succinic anhydride in the case of f) above.

2. The process according to Claim 1 wherein said reaction is carried out in the presence of an acid scavenger such as triethylamine.

## Claims (Claims for the following Contracting State(s): GR)

1. A process for preparing a compound selected from the group consisting of
a) N-ethoxycarbonyloxy-N-[1-(benzo[b]thien-2-yl)ethyl]urea;
b) N-methoxycarbonyloxy-N-[1-(benzo[b]thien-2-yl)ethyl]urea;
c) N-tert-butoxycarbonyloxy-N-[1-(benzo[b]thien-2-yl)ethyl]urea;
d) N-thioethylcarbonyloxy-N-[1-(benzo[b]thien-2-yl)ethyl]urea;
e) N-glutaryloxy-N-[1-(benzo[b]thien-2-yl)ethyl]urea; and
f) N-succinyloxy-N-[1-(benzo[b]thien-2-yl)ethyl]urea;
comprising reacting N-hydroxy-N-[1-(benzo[b]thien-2-yl)ethyl]urea with an N-hydroxy blocking group reagent which is
ethylchloroformate in the case of a) above;
methylchloroformate in the case of b) above;
di-tert-butylcarbonate in the case of c) above;
ethyl chlorothioformate in the case of d) above;
glutaric anhydride in the case of e) above; and
succinic anhydride in the case of f) above.

2. The process according to Claim 1 wherein said reaction is carried out in the presence of an acid scavenger such as triethylamine.

3. Use of a compound selected from the group consisting of
N-ethoxycarbonyloxy-N-[1-(benzo[b]thien-2-yl)ethyl]urea;
N-methoxycarbonyloxy-N-[1-(benzo[b]thien-2-yl)ethyl]urea;
N-tert-butoxycarbonyloxy-N-[1-(benzo[b]thien-2-yl)ethyl]urea;
N-thioethylcarbonyloxy-N-[1-(benzo[b]thien-2-yl)ethyl]urea;
N-glutaryloxy-N-[1-(benzo[b]thien-2-yl)ethyl]urea; and
N-succinyloxy-N-[1-(benzo[b]thien-2-yl)ethyl]urea;
for manufacturing a medicament for inhibiting lipoxygenase enzymes in a host mammal in need of such treatment.

## Patentansprüche (Patentansprüche für folgende(n) Vertragsstaat(en): AT, BE, CH, LI, DE, DK, FR, GB, IT, LU, NL, SE)

1. Verbindung, die aus der Gruppe gewählt ist, die aus folgendem besteht:
N-Ethoxycarbonyloxy-N-[1-(benzo[b]thien-2-yl)ethyl]harnstoff;
N-Methoxycarbonyloxy-N-[1-(benzo[b]thien-2-yl)ethyl]harn-stoff;
N-tert-Butoxycarbonyloxy-N-[1-(benzo[b]thien-2-yl)ethyl]harnstoff;
N-Thioethylcarbonyloxy-N-[1-(benzo[b]thien-2-yl)ethyl]harnstoff;
N-Glutaryloxy-N-[1-(benzo[b]thien-2-yl)ethyl]harnstoff; und
N-Succinyloxy-N-[1-(benzo[b]thien-2-yl)ethyl]harnstoff.

2. Pharmazeutische Zusammensetzung zur Inhibition von Lipoxygenaseenzymen, die eine therapeutisch wirksame Menge einer Verbindung nach Anspruch 1 in Kombination mit einem pharmazeutisch verträglichen Träger umfaßt.

3. Verbindung nach Anspruch 1 zur Verwendung zur Inhibition von Lipoxygenaseenzymen bei einem Säugerpatienten, der einer solchen Behandlung bedarf.

4. Verwendung einer Verbindung nach Anspruch 1 zur Herstellung eines Medikamentes zur Inhibition von Lipoxygenaseenzymen bei einem Säugerpatienten, der einer solchen Behandlung bedarf.

## Patentansprüche (Patentansprüche für folgende(n) Vertragsstaat(en): ES)

1. Verfahren zur Herstellung einer Verbindung, die aus der Gruppe gewählt ist, die aus folgendem besteht:
a) N-Ethoxycarbonyloxy-N-[1-(benzo[b]thien-2-yl)ethyl]harnstoff;
b) N-Methoxycarbonyloxy-N-[1-(benzo[b]thien-2-yl)ethyl]harnstoff;
c) N-tert-Butoxycarbonyloxy-N-[1-(benzo[b]thien-2-yl)ethyl]harnstoff;
d)N-Thioethylcarbonyloxy-N-[1-(benzo[b]thien-2-yl)ethyl]harnstoff;
e) N-Glutaryloxy-N-[1-(benzo[b]thien-2-yl)ethyl]harnstoff; und
f) N-Succinyloxy-N-[1-(benzo[b]thien-2-yl)ethyl]harnstoff;
wobei das Verfahren die Umsetzung von N-Hydroxy-N-[1-(benzo[b]thien-2-yl)ethyl]harnstoff mit einem Reagenz umfaßt, das die N-Hydroxygruppe blockiert, das heißt mit
Ethylchlorformat im Falle von a) oben;
Methylchlorformat im Falle von b) oben;
Di-tert-butylcarbonat im Falle von c) oben;
Ethylchlorthioformat im Falle von d) oben;
Glutarsäureanhydrid im Falle von e) oben; und
Bernsteinsäureanhydrid im Falle von f) oben.

2. Verfahren nach Anspruch 1, wobei die Reaktion in Gegenwart eines Säurefängers wie Triethylamin durchgeführt wird.

## Patentansprüche (Patentansprüche für folgende(n) Vertragsstaat(en): GR)

1. Verfahren zur Herstellung einer Verbindung, die aus der Gruppe gewählt ist, die aus folgendem besteht:
a) N-Ethoxycarbonyloxy-N-[1-(benzo[b]thien-2-yl)ethyl]harnstoff;
b) N-Methoxycarbonyloxy-N-[1-(benzo[b]thien-2-yl)ethyl]harnstoff;
c) N-tert-Butoxycarbonyloxy-N-[1-(benzo[b]thien-2-yl)ethyl]harnstoff;
d)N-Thioethylcarbonyloxy-N-[1-(benzo[b]thien-2-yl)ethyl]harnstoff;
e) N-Glutaryloxy-N-[1-(benzo[b]thien-2-yl)ethyl]harnstoff; und
f) N-Succinyloxy-N-[1-(benzo[b]thien-2-yl)ethyl]harnstoff;
wobei das Verfahren die Umsetzung von N-Hydroxy-N-[1-(benzo[b]thien-2-yl)ethyl]harnstoff, mit einem Reagenz umfaßt, das die N-Hydroxygruppe blockiert, das heißt mit
Ethylchlorformat im Falle von a) oben;
Methylchlorformat im Falle von b) oben;
Di-tert-butylcarbonat im Falle von c) oben;
Ethylchlorthioformat im Falle von d) oben;
Glutarsäureanhydrid im Falle von e) oben; und
Bernsteinsäureanhydrid im Falle von f) oben.

2. Verfahren nach Anspruch 1, wobei die Reaktion in Gegenwart eines Säurefängers wie Triethylamin durchgeführt wird.

3. Verwendung einer Verbindung, die aus der Gruppe gewählt ist, die aus folgendem besteht:
N-Ethoxycarbonyloxy-N-[1-(benzo[b]thien-2-yl)ethyl]harnstoff;
N-Methoxycarbonyloxy-N-[1-(benzo[b]thien-2-yl)ethyl]harn-stoff;
N-tert-Butoxycarbonyloxy-N-[1-(benzo[b]thien-2-yl)ethyl]harnstoff;
N-Thioethylcarbonyloxy-N-[1-(benzo[b]thien-2-yl)ethyl]harnstoff;
N-Glutaryloxy-N-[1-(benzo[b]thien-2-yl)ethyl]harnstoff; und
N-Succinyloxy-N-[1-(benzo[b]thien-2-yl)ethyl]harnstoff;
zur Herstellung eines Medikamentes zur Inhibition von Lipoxygenaseenzymen bei einem Säugerpatienten, der einer solchen Behandlung bedarf.

## Revendications (Revendications pour l'(les) Etat(s) contractant(s) suivant(s): AT, BE, CH, LI, DE, DK, FR, GB, IT, LU, NL, SE)

1. Composé choisi dans le groupe constitué par:
la N-éthoxycarbonyloxy-N-[1-(benzo[b]thién-2-yl)éthyl]urée;
la N-méthoxycarbonyloxy-N-[1-benzo[b]thién-2-yl)éthyl]urée;
la N-tert-butoxycarbonyloxy-N-[1-(benzo[b]thién-2-yl)éthyl]urée;
la N-thioéthylcarbonyloxy-N-[1-(benzo[b]thién-2-yl)éthyl]urée;
la N-glutaryloxy-N-[1-(benzo[b]thién-2-yl)éthyl]urée; et
la N-succinyloxy-N-[1-(benzo[b]thién-2-yl)éthyl]urée.

2. Composition pharmaceutique pour l'inhibition des enzymes lipoxygénases, comprenant une quantité thérapeutiquement efficace d'un composé selon la revendication 1 en combinaison avec un support pharmaceutiquement acceptable.

3. Composé selon la revendication 1 à utiliser pour l'inhibition d'enzymes lipoxygénases chez un mammifère hôte nécessitant un tel traitement.

4. Utilisation d'un composé selon la revendication 1 pour la fabrication d'un médicament destiné à inhiber les enzymes lipoxygénases chez un mammifère hôte nécessitant un tel traitement.

## Revendications (Revendications pour l'(les) Etat(s) contractant(s) suivant(s): ES)

1. Procédé de préparation d'un composé choisi dans le groupe constitué par:
a) la N-éthoxycarbonyloxy-N-[1-(benzo[b]thién-2-yl)éthyl]urée;
b) la N-méthoxycarbonyloxy-N-[1-(benzo[b]thién-2-yl)éthyl]urée;
c) la N-tert-butoxycarbonyloxy-N-[1-(benzo[b]thién-2-yl)éthyl]urée;
d) la N-thioéthylcarbonyloxy-N-[1-(benzo[b]thién-2-yl)éthyl]urée;
e) la N-glutaryloxy-N-[1-(benzo[b]thién-2-yl)éthyl]urée; et
f) la N-succinyloxy-N-[1-(benzo[b]thién-2-yl)éthyl]urée,
selon lequel on fait réagir la N-hydroxy-N-[1-(benzo[b]thién-2-yl)éthyl]urée avec un réactif de blocage du groupe N-hydroxy qui est le chloroformate d'éthyle dans le cas de a) ci-dessus;
le chloroformate de méthyle dans le cas de b) ci-dessus;
le carbonate de di-tert-butyle dans le cas de c) ci-dessus;
le chlorothioformate d'éthyle dans le cas de d) ci-dessus;
l'anhydride glutarique dans le cas de e) ci-dessus; et
l'anhydride succinique dans le cas de f) ci-dessus.

2. Procédé selon la revendication 1, dans lequel ladite réaction s'effectue en présence d'un accepteur d'acide comme la triéthylamine.

## Revendications (Revendications pour l'(les) Etat(s) contractant(s) suivant(s): GR)

1. Procédé de préparation d'un composé choisi dans le groupe constitué par:
a) la N-éthoxycarbonyloxy-N-[1-(benzo[b]thién-2-yl)éthyl]urée;
b) la N-méthoxycarbonyloxy-N-[1-(benzo[b]thién-2-yl)éthyl]urée;
c) la N-tert-butoxycarbonyloxy-N-[1-(benzo[b]thién-2-yl)éthyl]urée;
d) la N-thioéthylcarbonyloxy-N-[1-(benzo[b]thién-2-yl)éthyl]urée;
e) la N-glutaryloxy-N-[1-(benzo[b]thién-2-yl)éthyl]urée; et
f) la N-succinyloxy-N-[1-(benzo[b]thién-2-yl)éthyl]urée,
selon lequel on fait réagir la N-hydroxy-N-[1-(benzo[b]thién-2-yl)éthyl]urée avec un réactif de blocage du groupe N-hydroxy qui est le chloroformate d'éthyle dans le cas de a) ci-dessus;
le chloroformate de méthyle dans le cas de b) ci-dessus;
le carbonate de di-tert-butyle dans le cas de c) ci-dessus;
le chlorothioformate d'éthyle dans le cas de d) ci-dessus;
l'anhydride glutarique dans le cas de e) ci-dessus; et
l'anhydride succinique dans le cas de f) ci-dessus.

2. Procédé selon la revendication 1, dans lequel ladite réaction s'effectue en présence d'un accepteur d'acide comme la triéthylamine.

3. Utilisation d'un composé choisi dans le groupe constitué par:
la N-éthoxycarbonyloxy-N-[1-(benzo[b]thién-2-yl)éthyl]urée;
la N-méthoxycarbonyloxy-N-[1-(benzo[b]thién-2-yl)éthyl]urée;
la N-tert-butoxycarbonyloxy-N-[1-(benzo[b]thién-2-yl)éthyl]urée;
la N-thioéthylcarbonyloxy-N-[1-(benzo[b]thién-2-yl)éthyl]urée;
la N-glutaryloxy-N-[1-(benzo[b]thién-2-yl)éthyl]urée; et
la N-succinyloxy-N-[1-(benzo[b]thién-2-yl)éthyl]urée,
pour la fabrication d'un médicament destiné à inhiber les enzymes lipoxygénases chez un mammifère hôte nécessitant un tel traitement.
